# EUROPEAN PATENT SPECIFICATION

(11) **EP 0 253 467 B1**
(45) Date of publication and mention of the grant of the patent: **18.11.1993**
(21) Application number: 87303011.8
(22) Date of filing: 07.04.1987
(51) Int. Cl.: B01D 19/02, B01D 19/00, A61M 1/36

(54) **Liquid and gas separation system**
Einrichtung zum Trennen von Gasen und Flüssigkeiten
Système de séparation du liquide et du gaz

(30) Priority: 14.07.1986 US 885963
(43) Date of publication of application: 20.01.1988
(73) Proprietor: BAXTER INTERNATIONAL INC. (a Delaware corporation), Deerfield Illinois 60015 (US)
(72) Inventor: Gordon, Lucas Streator, Laguna Beach California 92651 (US)
(74) Representative: Orchard, Oliver John

(56) References cited:
- EP-A- 0 122 748
- DE-A- 1 949 038
- DE-A- 2 704 554
- DE-C- 895 441
- US-A- 4 568 367

## Description

This invention relates to a system useful for the separation of blood and gas, and more particularly to a medical device, such as a blood oxygenator or a cardiotomy reservoir, which employs a defoamer system that affords excellent separation of macroscopic and microscopic air from blood while at the same time minimizing blood path contact with the silicone containing compounds typically used in the defoamer.

The invention for which rights are claimed is limited to a defoaming device for use in, and a method for using the device in, separating foam, macroscopic and microscopic bubbles from blood, although it will be understood that the device may be used with other liquids.

There are many systems known in the art which are used to remove gas (such as air) from fluids and which also employ a defoamer assembly in such systems. For example, in various types of surgical procedures, it is often necessary to perform a treatment whereby the patients blood is subject to a bypass flow outside of the patients body, and an apparatus such as, an oxygenator is employed. In many such oxygenators, oxygen is transferred to the blood via a procedure which forms a foam and therefore requires a defoamer assembly.

These oxygenators are used in open-heart surgery and other operations and treatments of the body when it is necessary to establish an extracorporeal circulation system for temporarily assuming the functions of the heart and lungs of the patient. In such a system, the oxygenator operates to perform the function usually performed by the lungs of the patient, i.e., the life-supporting transfer of oxygen into the blood and carbon dioxide out of the blood. The oxygenator is used in association with a pump which performs the function of the heart to cause circulation of the blood. Thus, early versions of the oxygenator were often referred to as "heart-lung" machines. The early heart-lung machines were typically rotating discs which passed through a pool of blood, but were only partially immersed therein such that the free surface of the disc exposed the blood to oxygen and accomplished some gas transfer. After this, bag-type oxygenators were introduced which were superior to the disc oxygenators, but which left much to be desired.

At the present time two principle types of blood oxygenators are used which have proven highly efficient, provide minimal blood trauma, are convenient to set up and operate, are cost effective and have provided excellent clinical results, i.e. bubble oxygenators and membrane oxygenators. In a membrane oxygenator, a thin, highly gas permeable membrane is placed between the gas and blood. Venous blood flows along one side of the membrane and gas is on the other side. A pressure gradient is established so that when the partial pressure for oxygen is higher in the ventilating gas than the partial pressure for oxygen in the venous blood, oxygen will diffuse across the membrane into the blood. Bubble oxygenators simply diffuse gas bubbles into venous blood. The oxygenated blood is typically defoamed before it is ready for delivery to the patient.

In medical devices such as the oxygenators as described above, and in other medical devices such as cardiotomies and hardshell venous reservoirs, air or some other gas can be introduced into the blood, e.g. oxygen (in an oxygenator), nitrogen, carbon dioxide, etc. Typically, when this occurs it is medically necessary to remove certain gas from the blood prior to the blood going to the patient. Separation of the blood from the gas requires the medical device to be used in combination with a defoaming device which typically incorporates some sort of an agent to assist in breaking the foam down. In medical applications, about the only agent that has proved acceptable is a silicone antifoam agent. However, in the process of the silicone agent performing its job, i.e. remove the gas from the blood, a small amount of the silicone actually is transferred into the blood. The problem with this is that silicone is not metabolized by the human body, and therefore silicone accumulates in the body. Even though silicone is an inert material it is undesirable within the human body because it can tend to clog up some of the very small capillaries and arteries within the human body.

As described herein, the features of the present invention can be employed in various types of medical devices. Examples of the type of so-called membrane oxygenators which can employ the features of the present invention are described in U.S. Patent Numbers 4,094,792 and 4,196,075 both of which are assigned to Bentley Laboratories, Inc., the assignee of the present invention. In addition Bentley Laboratories, Inc. products identified as the Bentley BCM-3 and BCM-7 integrated membrane oxygenators which are oxygenators having three major components can incorporate the defoamer of the present invention. Examples of the bubble type blood oxygenator that can employ the features of the present invention are described in U.S. Patent Numbers 3,468,631, 3,488,158 and 3,578,411, the last two of which describe devices which have come to be known as the Bentley Oxygenator, and also U.S. Patent Numbers 4,282,180 and 4,440,723, both assigned to Bentley Laboratories, Inc.

Various prior art examples of blood oxygenators and gas-liquid type of transfer apparatus are described in U.S. Patent Numbers 3,065,748; 3,256,883; 3,493,347; 4,073,622; 4,138,288; 4,182,739; 4,203,944; 4,203,945; 4,288,125; 4,231,988; 4,272,373; 4,336,224; 4,370,151; 4,374,088; 4,396,584; 4,407,777; 4,440,722; 4,493,692 and 4,533,516.

In the specification of European Patent Application No. 122748, which was published on October 24, 1984, there is described a filter device for a cardiotomy reservoir which comprises multiple layers of concentric tubular elements with an inner annular inlet passage to which blood is supplied. A gross filter layer comprising an open cell blood compatible, synthetic polymeric foam material to collapse blood foam is preferably treated with an antifoam agent.

In the specification of German Patent No. 895,441, which was published in November, 1953, there is described a method of destroying foam on the surface of liquid in a vessel by arranging a silicone wetted surface at some distance above the surface and extending practically to the edge of the vessel. In the case of cylindrical vessels of small diameters, it is said that it is usually sufficient for the walls of the vessel above the surface of the liquid to be wetted with silicones.

It is an object of the present invention to provide a device which may be used for separating gas from blood and in which the above-noted disadvantages are minimised.

In an arrangement to be described there is a defoaming device for separating gas in the form of foam from blood and then breaking the foam down into blood and the gas. The device comprises a reservoir and a filtering element that does not contain an antifoaming agent and which is positioned in the lower portion of the reservoir to first contact the blood and separate gas foam and bubbles from the blood. Positioned in the reservoir above the maximum fluid level is an element containing an antifoaming agent which then comes in contact with the foam that rise from the filtering element and breaks down the foam.

Basically, when blood is moving through a medical device it may from time to time have some gas in it. When gas is in the blood, and it's desired to remove it, an antifoam agent such as a silicone material (e.g. simethicone) is typically used. However, it is preferred not to have the blood directly contact the silicone material. The defoamer device in accordance with the features of the present invention is unique in that when in operation, blood without gas will not contact the silicone containing material. Foam, macroscopic bubbles, and microscopic bubbles, appearing in the blood will be separated from the blood without contacting the silicone antifoam agent. Then, after separation, the foam and bubbles are allowed to travel to a portion of the defoamer assembly positioned above the maximum level of blood in the assembly's reservior where the foam and bubbles are then placed in contact with an antifoam dipped material. Thus, the only thing that contacts the antifoam agent is the foam or bubbles that rise up to the area above the blood level where the antifoam agent is located. The present invention is unique because it defines a defoamer which allows selective exposure of only the foam and bubbles to an antifoaming agent.

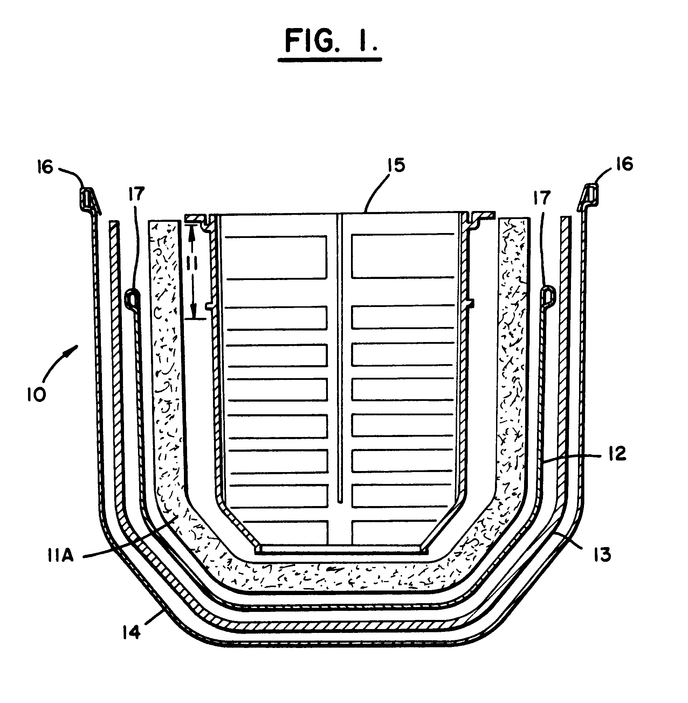

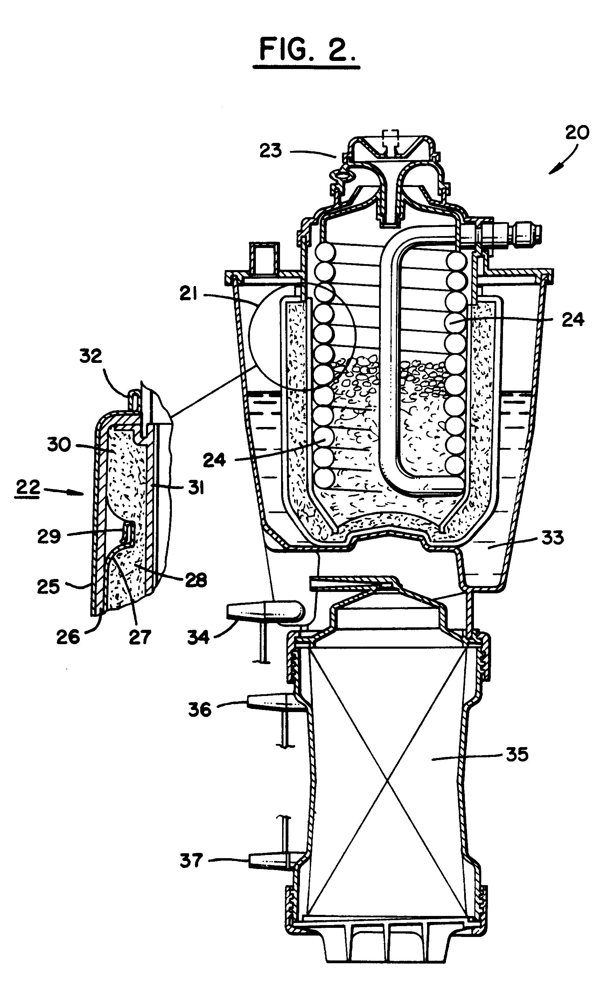

For a better understanding of the invention as well as other objects and further features thereof, reference is made to the following detailed disclosure taken in conjunction with the accompanying drawings wherein: FIG. 1 is an enlarged diagrammatic plan sectional view illustrating the principles of a defoaming apparatus and FIG. 2 is a plan sectional view of a membrane oxygenator and an enlarged portion thereof illustrating how a defoaming apparatus in accordance with the present invention can be used with the oxygenator.

Broadly speaking, the defoaming device to be described can be used for removing a gas, such as air, from various types of fluids, such as blood. The defoamer system as described herein is particularly suited for use in any open system device which requires air-blood separation. Excellent opportunities for using this defoamer system thereby exist in such medical devices as, for example, bubble oxygenators, membrane oxygenators, cardiotomies, hardshell venous reservoirs, blood autotransfusion systems and oxygenated blood cardioplegia systems. Thus, although the unique features of the defoaming device in accordance with the present invention will be described below with regard to its use in an oxygenator for the purpose of removing gas foam and bubbles from blood, it is to be understood that the defoaming device has broader use, does not require the particular structural features of the particular embodiment described herein and is capable of removing various gases from different liquids when used with a medical device and in other environments.

The basic features of a preferred embodiment of the defoaming device 10 of this invention are shown in Figure 1. The device illustrated can be identified as either the Bentley Laboratories, Inc. BCM-3 or BCM-7 defoamer which can be used, for example, in either the Bentley BCM-3 or BCM-7 integrated membrane oxygenator as described in commonly assigned and copending Serial No. , filed , (Docket Number MM:550) entitled "Integrated Membrane Oxygenator, Heat Exchanger and Reservoir or in the Bentley BMR-1500 membrane oxygenator reservoir. The defoamer device 10 is a three-piece assembly which affords excellent separation of foam and macroscopic and microscopic air from blood while minimizing blood path contact with the silicone containing antifoam compound that is employed in the device. The three primary components of the defoamer device 10 shown in Figure 1 are as follows: (a) A low antifoam dipped (only on the top portion thereof 11,) polyurethane, thermally reticulated (open cell), foam pre-stage 11A constructed, for example, from 12.7 mm. thick, 100 ppi (pores per inch) polyurethane; (b) a screen 12 which can be a heparin coated 50 micro meters (mesh opening in microns) monodur polyester screen having a 36 percent open area (twill weave); and (c) a foam spacer stage 13 which which can be a 3.175 mm. thick, 15 ppi polyurethane, thermally reticulated (open cell) foam spacer stage.

The polyurethane foam pre-stage 11 is the only element of defoamer device 10 which includes an antifoaming agent. In accordance with the features of this invention an antifoam compound (e.g. simethicone) is applied to the low antifoam defoamer element 11A only along a relatively narrow border 11, e.g. a 2.54 cm (two inch) border, measured from the top portion of defoamer 10 (Note, 11 and 11A are the same piece of foam material). It is this low antifoam design which presents an antifoam coated surface only to the target blood foam and bubbles which, since being buoyant, move to the top of the blood. Thus contact between the blood and the silicone coating antifoam agent is minimized i.e. contact occurs with the foam and bubbles and the antifoam agent above the maximum liquid surface level of the blood. When defoaming device 10 is employed in an oxygenator, during normal operation the maximum blood column height within the defoamer remains below the lower margin area of the element 11 which contains the antifoam material. Microbubbles are not significantly affected by the simethicone compound or other antifoaming agents and exposure of the whole blood path to the antifoaming agents is unnecessary. The defoaming device 10 allows selective exposure of only blood foam to an antifoaming agent.

The 100 ppi polyurethane foam pre-stage 11A acts to filter foam, macroscopic air bubbles and microscopic air bubbles appearing in the blood before its presentation to the heparin coated polyester microscreen 12. Although the microscreen 12 is capable of removing the macroscopic air which is first presented to the pre-stage polyurethane foam 11, doing so causes an occlusive air film to form on the screen. Exposure of microscreens to large amounts of macroscopic air decreases their effective surface area and can impede their ability to pass fluids, even when treated with wetting agents such as heparin. A possible consequence of air occlusion of these microscreens is that blood passing through the diminished screen area is subjected to increased flow resistance and pressure drop. These increases may produce blood formed elements damage. Placement of a polyurethane foam "filtering stage" prior to the heparin coated screen significantly improves the blood air elimination as well as the blood handling capability of the defoamer assembly.

After blood passes through the 100 ppi pre-stage 11, it comes into contact with the 50 micro meters heparin dipped polyester screen 12. This screen effectively eliminates microbubbles greater than 50 micro meters in diameter. Although polyester microscreen fabrics are available with smaller mesh openings which would provide even greater air filtration, these fabrics would present certain disadvantages. As the fabric's mesh opening size decreases, the percent open area of the fabric (the effective blood passage area) decreases significantly. As the percent open area decreases, the blood is subjected to greater resistances and shearing forces. Increased shearing forces are reflected in greatly increased blood damage. The 50 micro meters PES twill heparin dipped screen provides excellent microbubble filtration in concert with a relatively high 36 percent open area. In-vitro evaluation of prolonged blood flow through this screen has clearly demonstrated the excellent blood handling capability of this material.

As illustrated in Figure 1, the heparin coated microscreen 12 does not enclose the upper portion of the polyurethane foam 11. During normal operation of the defoaming device 10, the blood level within the defoamer will always be below the margin of the polyester microscreen. Should the screen 12 become occluded, the blood level within the defoamer will exceed the height of the screen and blood flow will bypass the screen. All blood flow will pass through the antifoam treated section of the polyurethane defoamer 11 and cascade over the occluded microscreen. This integral bypass feature of the defoamer assembly 10 is a crucial safety feature which allows the use of a microscreen in the blood path without the threat of total blood path occlusion concommitant with screen occlusion.

The third element of the defoamer assembly 10 is a spacer stage 13 which is preferably a 3.175 mm thick 15 ppi polyurethane, thermally reticulated (open cell) material. This element is a spacer stage which prevents the wicking of blood between the polyester microscreen 12 (or the upper segment of the 100 ppi prestage 11) and the outer containment layer, e.g. a polyester tricot stock, in which the elements of the defoamer assembly are enclosed. A large pore material, such as a 15 ppi polyurethane material 13 is preferably used for this spacer stage to limit material surface area for air and blood remixing.

All three elements 11A, 12, and 13 are preferably located in a polyester tricot sock 14 outer player and the entire defoaming assembly is secured onto a rigid vented support grid 15. The sock 14 and screen element 12 are connected to straps at positions 16 and 17 in the manner as described herein below, i.e. the example given of how the defoaming assembly 10 can be used in a membrane oxygenator.

In accordance with the features of the particular embodiment of the defoamer assembly that has been described herein above, there are certain critical parameters which will enable the defoamer assembly to operate in the environment of an oxygenator, particularly a membrane oxygenator, in an efficient manner. In defoamer assembly 10, the polyurethane foam 11, 11A and 13 and polyester screen 12 pore sizes are critical to the efficient functioning of the entire assembly.

In the polyurethane thermally reticulated foam prestage elements 11 and 11A, a preferred pore size for the material is about 100 ppi and this pore size can range from about 80 to 110 ppi. It is preferred not to use foam for element 11 having a pore size less than about 80 ppi because this type of material will not adequately screen macroscopic air. If the pore size of element 11A is greater than about 110 ppi it may have too high a breakthrough volume thereby bringing the blood path in contact with antifoaming agents. In the monodur polyester screen 12, a preferred pore size for the screen material is about 50 micro meters and can range from about 50 to 71 micro meters. It is preferred not to use a screen having less than about 50 micro meters openings because this type of microscreen has an inadequate percent of open area. A low percent of open area may produce unacceptable levels of blood damage. If the screen has greater than about 71 micro meters openings, it will exhibit little affect in reducing microbubble levels. In the polyurethane thermally reticulated foam spacer stage 13, a preferred pore size is about 15 ppi and this can range from about 15 to 25 ppi. It is preferred not to use a foam spacer stage 13 having a ppi call out less than about 15 ppi because it is difficult to manufacture defoamer material with this low pore size. Polyurethane foam material with a ppi call out greater than about 25 ppi is generally not acceptable because it presents too much material surface area for air/blood remixing.

The operation and use of the defoaming assembly as described herein above will now be described in the environment of a membrane oxygenator. As illustrated in Figure 2, there is shown a membrane oxygenator 20 with the circled area 21 illustrating in an enlarged manner the structure of a defoaming assembly 22 incorporating the unique features of the present invention. In use, blood from a patient is fed into the membrane oxygenator through a venous inlet connector located at the top portion 23 of the oxygenator. Once in the oxygenator, the blood slowly trickles in a downward direction onto the heat exchanger tubes 24, which are preferably in the form of a helically wrapped tube, and then into the defoaming assembly 22. It should be noted that in the membrane oxygenator the purpose of passing the blood through the defoaming assembly is to separate incidental air that is fed into the oxygenator along with the blood.

The defoaming assembly 22 is structured of an outer layer of a polyester tricot sock 25. Located adjacent sock 25 is a layer 26 of a 15 ppi polyurethane foam material. A 50 micro meters modur polyester screen 27 is positioned between foam material 26 and layer 28 of 100 ppi polyurethane foam material. A strap member (not shown) is secured to screen 27 at position 29 and used to squeeze foam layer 28 inwardly (as shown) at the approximate location of the maximum blood level within the defoaming assembly. The 100 ppi polyurethane foam material 30 positioned above this indented portion of the 100 ppi foam (i.e. positioned above the maximum surface of the blood level within the defoaming assembly) is the portion of the 100 ppi foam material which includes an antifoaming agent such as, for example, simethicone. The next illustrated layer of defoaming assembly 22 represents a support grid 31. Another strap element is secured to the sock 25 at position 32 and holds the sock onto the support grid.

During use of the membrane oxygenator 20, the blood travels from the heat exchange tubes 24 and is directed into contact with the 100 ppi polyurethane foam pre-stage 28 (that part of the 100 ppi foam without the antifoaming agent) which acts to filter foam, macroscopic air bubbles, and microscopic air bubbles appearing in the blood. Afterwards the blood travels to the heparin coated polyester microscreen 27 which effectively eliminates the microbubbles of air in the blood greater than 50 micro meters in diameter. The microbubbles separated from the blood and any other foam travel to the antifoam area 30 of the 100 ppi polyurethane foam which is located above the maximum surface level of the blood when the separation of the air from blood occurs. Thus, the defoamer allows selective exposure of only blood foam (bubbles) to the antifoaming agent.

After the blood passes through the defoaming assembly 22, it goes into a venous reservoir 33. Thereafter, the blood passes from the reservoir out of a venous reservoir outlet connector 34 to a pump (not shown) which pumps the blood back to the lower portion of oxygenator 20 through an oxygenator inlet connector (not shown). It is in the lower portion of the membrane oxygenator where oxygen transfer to the blood actually occurs. After being pumped back into the oxygenator, the blood flows thru a plurality of vertical hollow fibers 35. The oxygenator includes an oxygen inlet connector 37 which feeds the oxygen to fibers 35 and an oxygen outlet connector 36 which allow the oxygen to travel out of the oxygenator. Basically the blood flows through the fibers 35 while the oxygen flows around the hollow fibers whereby the oxygenation takes place.

The apparatus described in accordance with the present invention can be used to defoam numerous types of liquids. One example of a liquid is blood, which has been used as a specific example to described a detailed embodiment of the present invention.

Although the invention has been described with reference to a particular embodiment by way of example, it will be understood that variations and modifications thereof, as well as other embodiments may be made within the scope of the appended claims.

## Claims

1. A defoaming device for use in separating foam, macroscopic and microscopic bubbles from blood, including a reservoir (33) for containing the blood and having inflow means (23) and outflow means (34) for the blood communicating with the reservoir (33), a first filter (11A) (28) which is positioned to contact blood that is introduced through the inflow means (23) and to separate foam, macroscopic and microscopic air bubbles therefrom, and a screen (12) (27) through which blood exiting from the first filter (11A) (28) passes, and a second filter (13) (26) for receiving blood exiting from the screen (12) (27), characterised in that an upper portion (11) (30) of the first filter (11A) (28) contains an anti-foaming agent and is at a predetermined height above an anticipated maximum level of blood in the reservoir (33) such that direct contact between the upper portion (11) (30) and the blood at or below the maximum level is substantially avoided, and in that the remainder of the first filter (11A) (28) does not contain an anti-foaming agent, whereby the upper portion (11) (30) of the first filter (11A) (28) receives foam, macroscopic bubbles and microscopic bubbles from the blood as it flows through the remainder of the first filter (11A) (28) and the screen (12) (27).

2. A defoaming device as claimed in Claim 1 characterised in that the screen (12) (27) extends only over the said remainder of the first filter (11A) (28), whereby should the level of the blood in the reservoir (33) exceed the anticipated maximum level it will bypass the screen (12) (27).

3. A defoaming device as claimed in either Claim 1 or Claim 2 characterised in that the screen (12) (27) is treated with a wetting agent.

4. A defoaming device as claimed in any one of Claims 1 to 3 characterised in that the anti-foam agent contains silicone.

5. A defoaming device as claimed in any one of the preceding claims characterised in that the second filter (13) (26) is formed of a polyurethane foam material.

6. A method of operating a defoaming device as claimed in any one of the preceding claims characterised in that in operation the level of the blood is maintained such that direct contact between the upper portion (11) (30) and the blood at or below the maximum level is substantially avoided, and in that the upper portion (11) (30) of the first filter (11A) (28) receives foam, macroscopic bubbles and microscopic bubbles from the blood as it flows through the remainder of the first filter (11A) (28) and the screen (12) (27).

## Patentansprüche

1. Entschäumungsvorrichtung zur Verwendung beim Abscheiden von Schaum, makroskopischen und mikroskopischen Blasen aus Blut, die aufweist: ein Reservoir (33), um das Blut zu enthalten, das eine Zuflußeinrichtung (23) und eine Abflußeinrichtung (34) für das Blut hat, die mit dem Reservoir (33) in Verbindung stehen, einen ersten Filter (11A) (28), der positioniert ist, um mit Blut in Kontakt zu gelangen, das durch die Zuflußeinrichtung (23) eingeleitet wird, und um Schaum, makroskopische und mikroskopische Luftblasen daraus abzuscheiden, und ein Sieb (12) (27), durch das aus dem ersten Filter (11A) (28) austretendes Blut hindurchtritt, und einen zweiten Filter (13) (26), um aus dem Sieb (12) (27) austretendes Blut aufzunehmen, dadurch gekennzeichnet, daß ein oberer Bereich (11) (30) des ersten Filters (11A) (28) ein Schaumverhütungsmittel enthält und sich auf einer vorbestimmten Höhe über einem erwarteten Maximalblutpegel in dem Reservoir (33) befindet, so daß direkter Kontakt zwischen dem oberen Bereich (11) (30) und dem Blut auf oder unter dem Maximalpegel im wesentlichen vermieden wird, und daß der Rest des ersten Filters (11A) (28) kein Schaumverhütungsmittel enthält, wodurch der obere Bereich (11) (30) des ersten Filters (11A) (28) Schaum, makroskopische Blasen und mikroskopische Blasen aus dem Blut aufnimmt, während es durch den Rest des ersten Filters (11A) (28) und das Sieb (12) (27) fließt.

2. Entschäumungsvorrichtung nach Anspruch 1, dadurch gekennzeichnet, daß sich das Sieb (12) (27) nur über den genannten Rest des ersten Filters (11A) (28) erstreckt, wodurch das Blut das Sieb (12) (27) umgeht, falls der Pegel des Bluts in dem Reservoir (33) den erwarteten Maximalpegel überschreitet.

3. Entschäumungsvorrichtung nach Anspruch 1 oder 2, dadurch gekennzeichnet, daß das Sieb (12) (27) mit einem Netzmittel behandelt ist.

4. Entschäumungsvorrichtung nach einem der Ansprüche 1 bis 3, dadurch gekennzeichnet, daß das Schaumverhütungsmittel Silicon enthält.

5. Entschäumungsvorrichtung nach einem der vorhergehenden Ansprüche, dadurch gekennzeichnet, daß der zweite Filter (13) (26) aus einem Polyurethanschaumstoff gebildet ist.

6. Verfahren zum Betreiben einer Entschäumungsvorrichtung nach einem der vorhergehenden Ansprüche, dadurch gekennzeichnet, daß im Betrieb der Pegel des Bluts derart unterhalten wird, daß direkter Kontakt zwischen dem oberen Bereich (11) (30) und dem Blut auf oder unter dem Maximalpegel im wesentlichen vermieden wird, und daß der obere Bereich (11) (30) des ersten Filters (11A) (28) Schaum, makroskopische Blasen und mikroskopische Blasen aus dem Blut aufnimmt, während es durch den Rest des ersten Filters (11A) (28) und das Sieb (12) (27) fließt.

## Revendications

1. Dispositif d'émulsifiant utilisable pour séparer du sang l'émulsion, les bulles macroscopiques et microscopiques, comprenant un réservoir (33) contenant le sang des organes d'entrée (23) et de sortie (34) pour le sang et communiquant avec le réservoir (33), un premier filtre (11A, 28) qui est disposé pour être en contact avec le sang qui est introduit à travers les organes d'entrée (23) et pour y séparer l'émulsion, les bulles d'air macroscopiques et microscopiques, un tamis (12, 27) à travers lequel passe le sang sortant du premier filtre (11A, 28), et un deuxième filtre (13, 26) recevant le sang sortant du tamis (12, 27), caractérisé en ce qu'une partie supérieure (11, 30) du premier filtre (11A, 28) contient un agent anti-émulsifiant et se trouve à une hauteur prédéterminée au-dessus d'un niveau maximum envisagé du sang dans le réservoir (33), de sorte qu'on élimine sensiblement un contact direct entre la partie supérieure (11, 30) et le sang au ou en-dessous du niveau maximum, et en ce que le reste du premier filtre (11A, 28) ne contient pas d'agent anti-émulsifiant, de façon que la partie supérieure (11, 30) du premier filtre (11A, 28) reçoive i'émulsion, les bulles macroscopiques et les bulles microscopiques du sang lorsqu'il s'écoule à travers le reste du premier filtre (11A, 28) et le tamis (12, 27).

2. Dispositif d'émulsifiant, selon la revendication 1, caractérisé en ce que le tamis (12, 27) s'étend seulement au-dessus du reste du premier filtre (11A, 28), de façon que si le niveau du sang dans le réservoir (33) excède le niveau maximum envisagé, le sang contourne le tamis (12, 27).

3. Dispositif d'émulsifiant, selon l'une des revendications 1 ou 2, caractérisé en ce que le tamis (12, 27) est traité par un agent mouillant.

4. Dispositif d'émulsifiant, selon l'une des revendications 1 à 3, caractérisé en ce que l'agent anti-émulsifiant contient de la silicone.

5. Dispositif d'émulsifiant, selon l'une quelconque des revendications précédentes, caractérisé en ce que le deuxième filtre (13, 26) est constitué par une mousse de polyuréthane.

6. Procédé de fonctionnement d'un dispositif d'émulsifiant tel que revendiqué dans l'une des revendications précédentes, caractérisé en ce qu'en fonctionnement, le niveau du sang est maintenu de sorte qu'un contact direct entre la partie supérieure (11, 30) du premier filtre (11A, 28) reçoive l'émulsion, les bulles macroscopiques et les bulles microscopiques du sang lorsqu'il s'écoule à travers le reste du premier filtre (11A, 28) et le tamis (12, 27).
